# EUROPEAN PATENT APPLICATION

(11) **EP 3 756 677 A1**
(43) Date of publication of application: **30.12.2020**
(21) Application number: 19757733.1
(22) Date of filing: 20.02.2019
(51) Int. Cl.: A61K 35/28, A61K 35/35, A61K 45/00, A61P 15/10

(54) **ERECTILE DYSFUNCTION THERAPEUTIC AGENT**

(30) Priority: 23.02.2018 JP 2018031340
(71) Applicant: Meis Technology Inc., Nagoya-shi, Aichi 4640858 (JP)
(72) Inventor: MATSUMOTO Seiji, Asahikawa-shi, Hokkaido 078-8510 (JP); YAMAMOTO Tokunori, Asahikawa-shi, Hokkaido 078-8510 (JP); HOTTA Yuji, Nagoya-shi, Aichi 464-0074 (JP); KIMURA Kazunori, Nagoya-shi, Aichi 466-0051 (JP)
(74) Representative: Steinecke, Peter
(86) International application number: PCT/JP2019/006205
(87) International publication number: WO 2019/163798

(57) **Abstract**

An object of the present invention is to provide a novel therapeutic strategy for erectile dysfunction. Disclosed is a therapeutic agent for erectile dysfunction, including a filtrate obtained by filtering a disrupted solution of adipose tissue-derived stem cells or bone marrow-derived stem cells.

## Description

### [Technical Field]

The present invention relates to a therapeutic agent for erectile dysfunction (ED) and its use. The present application claims priority based on Japanese Patent Application No. 2018-031340 filed on February 23, 2018, the entire contents of which are incorporated herein by reference.

### [Background Art]

"Erectile dysfunction (ED)" is one kind of male sexual functional disorders and refers to "a condition that satisfactory sexual intercourse cannot be performed because sufficient erection is not attained during sexual intercourse or sufficient erection cannot be maintained". Erectile dysfunction (hereinafter also called "ED") is also called "erectile function disorder" or "erectile disorder". ED is classified into a mild type, a moderate type, and a complete type according to its severity. Further, ED is roughly divided into organic (caused by arterial sclerosis, nerve damage, etc.), psychogenic (caused by mental stress), and mixed (generated by combining both elements of the organic factor and the psychogenic factor) ED, according to causes.

With the advent of an aging society, the morbidity of erectile dysfunction is on the rise. The ED morbidity is 152 million in 1995, and is expected to increase to 322 million by 2025 (NPL 1). For erectile dysfunction, treatments such as drug therapy (PDE-5 inhibitor. For example, see PTLs 1 to 3) and psychotherapy, as well as surgical operation, are performed. There are many cases in which PDE-5 inhibitors are ineffective, and there is currently no other effective drug. Overseas, cavernosal injection (local injection) of prostaglandin (PG) E1 is carried out in cases in which PDE-5 inhibitors are ineffective, and the like, but the effect is limited. Moreover, although there are reports on effectiveness of stem cell therapy overseas, there is a risk of canceration. Albersen, M et al. have reported that, by adipose-derived stem cells (ADSC) and its lysate, improvement of erectile function was observed in a cavernous nerve injury model (NPL 2).

### [Citation List]

### [Patent Literature]

[PTL 1] WO 98/053819 A
[PTL 2] WO 00/033845 A
[PTL 3] WO 99/43674 A

### [Non Patent Literature]

[NPL 1] Ayta IA, McKinlay JB, Krane RJ. The likely worldwide increase in erectile dysfunction between 1995 and 2025 and some possible policy consequences. BJU Int 1999; 84:50-56.
[NPL 2] Albersen M, Fandel TM, Lin G, Wang G, Banie L, Lin CS, Lue TF. Injections of adipose tissue-derived stem cells and stem cell lysate improve recovery of erectile function in a rat model of cavernous nerve injury. J Sex Med 2010; 7(10): 3331-40.

### [Summary of Invention]

### [Technical Problem]

Currently, the first-line treatment for ED is administration of PDE-5 inhibitors, but due to various causes of ED, there are many cases in which a sufficient therapeutic effect cannot be obtained, and it cannot be used in some cases from the point of side effects and contraindications. Therefore, there remains a great need for highly effective new therapies. In order to meet the demand, an object of the present invention is to provide a novel therapeutic strategy for ED.

### [Solution to Problem]

In order to solve the above problems, the present inventors have focused on adult stem cells (also called tissue stem cells and somatic stem cells) and examined their effectiveness in detail. As a result, in two types of ED model animals (vascular ED model and diabetic ED model), a "filtrate" obtained by filtering a disrupted solution of adipose tissue-derived stem cells (called Adipose-derived stem cells: ASC, Adipose-derived regeneration cells: ADRC, Adipose-derived mesenchymal stem cells: AT-MSC, AD-MSC, etc.) showed an excellent therapeutic effect. On the other hand, in a neurogenic ED model, a filtrate similarly prepared from bone marrow-derived stem cells (BM-MSC) showed an excellent therapeutic effect. Moreover, when ultrasonic treatment was adopted as a means for preparing a disrupted cell solution, it was found that the therapeutic effect of the filtrate is enhanced as compared with the case of adopting freeze-thaw treatment.

The above results show that the filtrate prepared from ADS or BM-MSC (filtered disrupted cell solution) is effective as an ED therapeutic agent and has a wide range of application. In addition, the significance of the fact that the filtrate prepared from the disrupted cell solution, rather than the cells themselves, showed medicinal effects is extremely large, in consideration of clinical advantages such as it is not necessary to start cell culture while judging the timing of use, it is easy to prepare and handle the filtrate, the preparation time at the time of use can be shortened because the material (that is, ASC or BM-MSC) can be prepared in advance, and further, treatment with less concern of side effects becomes possible.

By the way, as described above, Albersen M et al. have reported that a lysate of adipose-derived stem cells (ADSC) had an erectile function-improving effect on a cavernous nerve injury model. The cell lysate used by Albersen M et al. is prepared by disrupting cells using osmotic pressure, repeating freeze-thaw treatment three times, and removing unnecessary substances by centrifugation. In contrast, the filtrate used by the present inventors is obtained by disrupting cells (ASC or BM-MSC) by freeze-thaw treatment or ultrasonic treatment, followed by centrifugation, and filtering the resulting supernatant through a filter. It is clearly distinguished from the cell lysate of Albersen M et al., particularly, in that cell debris and other contaminants are more reliably removed by filtering. Also, Albersen M et al. does not refer to cells other than ADSC (ASC), and the model used in the experiment (experimental system) is also limited to the cavernous nerve injury model.

The following inventions have been completed mainly based on the above achievements and consideration.
[1] A therapeutic agent for erectile dysfunction, including a filtrate obtained by filtering a disrupted solution of adipose tissue-derived stem cells or bone marrow-derived stem cells.
[2] The therapeutic agent for erectile dysfunction according to [1], wherein the disrupted solution is centrifuged before the filtering, and the obtained supernatant is filtered.
[3] The therapeutic agent for erectile dysfunction according to [1] or [2], wherein the disrupted solution is obtained by ultrasonic treatment.
[4] The therapeutic agent for erectile dysfunction according to any one of [1] to [3], which is used for treating organic erectile disorder or mixed erectile disorder.
[5] The therapeutic agent for erectile dysfunction according to [4], wherein the organic erectile disorder is neurogenic, vascular or diabetic erectile disorder.
[6] The therapeutic agent for erectile dysfunction according to any one of [1] to [5], which is used in combination with a PDE-5 inhibitor and/or a prostaglandin preparation.
[7] A method for producing a therapeutic agent for erectile dysfunction, including following steps (1) to (3):
   (1) disrupting adipose tissue-derived stem cells or bone marrow-derived stem cells;
   (2) obtaining a filtrate by filtering a disrupted solution obtained in step (1), or a supernatant obtained by centrifuging the disrupted solution; and
   (3) formulating the filtrate obtained in step (2).
[8] The production method according to [7], wherein step (1) is performed by ultrasonic treatment.
[9] A method for treating erectile dysfunction, including administering the therapeutic agent for erectile dysfunction according to any one of [1] to [5], to the corpus cavernosum penis, the corpus spongiosum penis, the external urethral sphincter or under the urethral mucosa of the external urethral sphincter part of a patient with erectile dysfunction.
[10] The treatment method according to [9], wherein a PDE-5 inhibitor and/or a prostaglandin preparation is co-administered.

### [Brief Description of Drawings]

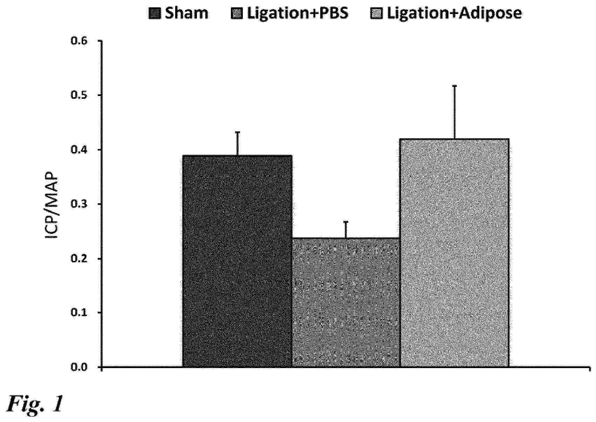

[Fig. 1] Effect of stem cell filtrate on vascular ED. Improvement of erectile function is observed by administration of adipose stem cell (ASC) filtrate. Sham: control group, Ligation + PBS: vascular ED + PBS administration group, Ligation + Adipose: vascular ED + ASC filtrate administration group. For each group, n = 3.

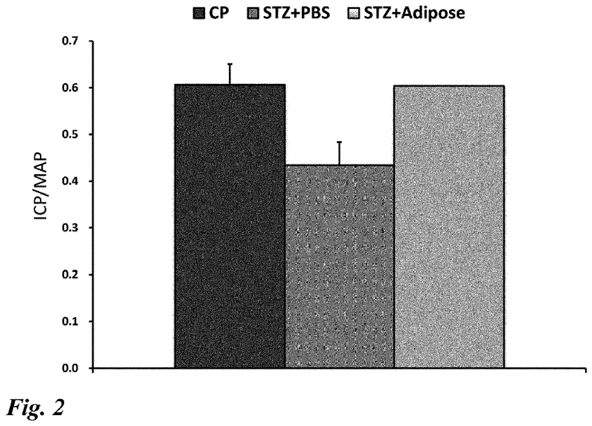

[Fig. 2] Effect of stem cell filtrate on diabetic ED. Improvement of erectile function is observed by administration of adipose stem cell (ASC) filtrate. CP: control group (n = 3), STZ + PBS: diabetic ED + PBS administration group (n = 3), STZ + Adipose: diabetic ED + ASC filtrate administration group (n = 2).

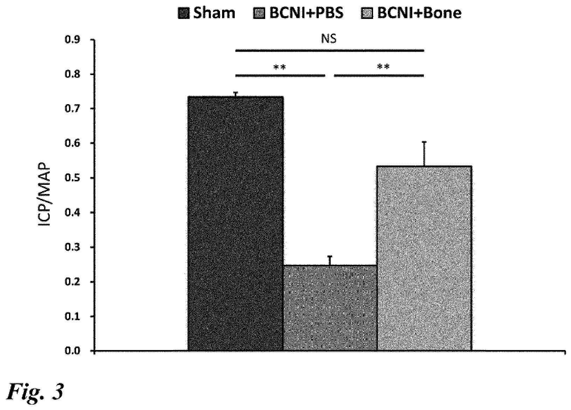

[Fig. 3] Effect of stem cell filtrate on neurogenic ED. Improvement of erectile function is observed by administration of bone marrow-derived stem cell (BM-MSC) filtrate. Sham: control group, BCNI + PBS: neurogenic ED + PBS administration group, BCNI + Bone: neurogenic ED + BM-MSC filtrate administration group. For each group, n = 3. By ANOVA and Bonferroni multiple t test. **P<0.01

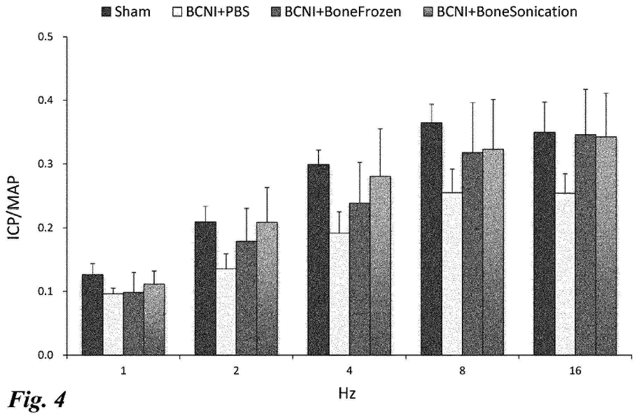

[Fig. 4] Effect of stem cell filtrate prepared by using non-freezing disruption (ultrasonic disruption) (neurogenic ED model). Sham: control group, BCNI + PBS: neurogenic ED + PBS administration group, BCNI + BoneFoezn: Neuronal ED + BM-MSC filtrate (freeze-thaw disruption) group, BCNI + BoneSonication: neurogenic ED + BM-MSC filtrate (ultrasonic disruption) group. For each group, n = 3.

### [Description of Embodiments]

The present invention relates to a therapeutic agent for erectile dysfunction (hereinafter, also called "the therapeutic agent of the present invention"). The therapeutic agent of the present invention is used for treating or preventing erectile dysfunction (ED). The "therapeutic agent" refers to a medicine that exhibits a therapeutic or preventive effect on a target disease (ED). The therapeutic effect includes relief (alleviation) of symptoms (pathological conditions) or concomitant symptoms characteristic of the target disease, prevention or delay of deterioration of symptoms, and the like. The latter can be regarded as one of the preventive effects in terms of preventing aggravation. In this way, the therapeutic effect and the preventive effect are partially overlapping concepts, and it is difficult to clearly distinguish them, and a practical benefit of doing so is small. A typical preventive effect is to prevent or delay recurrence of characteristic symptoms of the target disease. In addition, as long as it shows some therapeutic effect or preventive effect, or both, on the target disease, it falls under a therapeutic agent for the target disease.

In the therapeutic agent of the present invention, a filtrate obtained by filtering a disrupted solution of adipose tissue-derived stem cells (ASC) or bone marrow-derived stem cells (BM-MSC) (in other words, an extract obtained by filtering a disrupted cell solution of ASC or BM-MSC through a filter) is used, and the components contained therein bring about a unique effect, that is, improvement of erectile function.

Typically, the therapeutic agent of the present invention contains a filtrate obtained by filtering a disrupted solution obtained by disrupting adipose tissue-derived stem cells (ASC) or bone marrow-derived stem cells (BM-MSC). However, an insoluble component may be removed by centrifuging the disrupted solution before the filtering. That is, one (filtrate) obtained by filtering a supernatant obtained by centrifuging the disrupted cell solution may be used. The conditions for the centrifugation are, for example, 200 to 300 g for 5 to 10 minutes.

For example, a cell suspension prepared at a concentration of 1 × 10⁶ cells/ml to 1 × 10⁷ cells/ml is used for disruption treatment. In order to obtain a disrupted solution of ASC or BM-MSC, the ASC or BM-MSC may be subjected to disruption treatment, for example, freeze-thaw treatment (treatment of freezing and then thawing), ultrasonic treatment, treatment with a French press or a homogenizer, or the like. The cells may be disrupted by a non-physical treatment. Moreover, the cells to be subjected to the disruption treatment are not limited to living cells, and dead cells or damaged cells may be used. Among various disruption treatments, the freeze-thaw treatment is particularly preferable because it is simple and can avoid contamination caused by contact between an instrument and cells, which is sanitary. The freeze-thaw treatment may be repeated a plurality of times (for example, 2 to 5 times). Freezing conditions in the freeze-thaw treatment are not particularly limited, but for example, freezing may be performed at -20°C to -196°C. Thawing conditions are also not particularly limited. For example, thawing in warm water (for example, 35°C to 40°C), thawing at room temperature and the like can be adopted.

On the other hand, when ultrasonic treatment is adopted, improvement of the therapeutic effect can be expected, as evidenced by the examples described later. That is, the ultrasonic treatment can be said to be an effective disruption treatment for obtaining a therapeutic drug having a high therapeutic effect. An example of ultrasonic treatment conditions is treatment (repeating 10 seconds of disruption and 20 seconds of rest) at an output of 200 W to 300 W for 30 minutes.

Unnecessary components are removed by filtering. Further, by using a filter with an appropriate pore size, it is possible to remove unnecessary components and perform sterilization at the same time. The material, pore size and the like of the filter used for the filtering are not particularly limited. However, cellulose acetate can be exemplified as a preferable material. A metal filter may be used. Examples of the pore size are 0.2 µm to 0.45 µm.

The therapeutic agent of the present invention may contain other pharmaceutically acceptable ingredients such as carrier, excipient, disintegrant, buffer, emulsifier, suspension, soothing agent, stabilizer, preservative, antiseptic, and physiological saline.

The origin of ASC or BM-MSC used in the therapeutic agent of the present invention, that is, the biological species is not limited, but human cells are preferably used in consideration of a problem of immune rejection and the like.

As is also clear from the above description, the therapeutic agent of the present invention can be produced by the following steps (1) to (3):
(1) disrupting adipose tissue-derived stem cells or bone marrow-derived stem cells;
(2) obtaining a filtrate by filtering the disrupted solution obtained in step (1), or a supernatant obtained by centrifuging the disrupted solution; and
(3) formulating the filtrate obtained in step (2).

The cells (ASC or BM-MSC) used in step (1) may be prepared by a conventional method. ASC and BM-MSC are widely used for various purposes, and can be easily prepared by those skilled in the art with reference to literatures and books. Cells distributed from a public cell bank, commercially available cells and the like may be used. Hereinafter, an ASC preparation method (one example) will be described as an example of a cell preparation method.

### <Preparation method of ASC>

"The adipose tissue-derived stem cells (ASC)" in the present invention refers to somatic stem cells that are contained in an adipose tissue, and cells that are obtained by culture of the somatic stem cells (including subculture) also correspond to "the adipose tissue-derived stem cells (ASC)" as long as such cells maintain multipotency. Generally, ASC is obtained from an adipose tissue separated from a living body as a starting material, and prepared into "an isolated state" as a cell that constitutes a cell population (containing cells except for ASC, which are originated from the adipose tissue). "An isolated state" herein means that ASC is present in a state of being taken out from its original environment (that is, a state of constituting a part of a living body), in other words, a state of being different from an original state of its existence due to artificial manipulation. Note that adipose tissue-derived mesenchymal stem cells are also called ADRC (adipose-derived regeneration cells), AT-MSC (adipose-derived mesenchymal stem cells), AD-MSC (adipose-derived mesenchymal stem cells), and so on. In the present specification, the following terms, that is, adipose tissue-derived mesenchymal stem cells, ASC, ADRC, AT-MSC, and AD-MSC are used exchangeably.

ASC is prepared through steps such as separation of stem cells from a fat substrate, washing, concentration, and culture. A preparation method of ASC is not particularly limited. For example, ASC can be prepared according to, for example, known methods (Fraser JK et al. (2006), Fat tissue:an underappreciated source of stem cells for biotechnology. Trends in Biotechnology; Apr; 24(4):150-4. Epub 2006 Feb 20. Review.; Zuk PA et al. (2002), Human adipose tissue is a source of multipotent stem cells. Molecular Biology of the Cell; Dec; 13(12):4279-95.; Zuk PA et al. (2001), Multilineage cells from human adipose tissue:implications for cell-based therapies. Tissue Engineering; Apr; 7(2):211-28., and the like are served as references). Further, a device for preparing ASC from adipose tissues (for example, Celution (registered trademark) device (Cytori Therapeutics, Inc., USA, San Diego)) is also commercially available and ASC may be prepared using the device. When the device is used, cells that are cell surface marker CD29 and CD44 positive can be separated from adipose tissues. Specific examples of a preparation method of ASC are shown below.

### (1) Preparation of population of cells from adipose tissue

Adipose tissue can be obtained from an animal by means such as excision and suck. The term "animal" herein includes human and non-human mammalians (pet animals, domestic animal, and experimental animal. Specifically examples include monkey, pig, cattle/cow, horse, goat, sheep, dog, cat, mouse, rat, guinea pig, hamster, and the like). In order to avoid the problem of immunological rejection, it is preferable that adipose tissue is collected from the subject (recipient) to which the agent of the present invention is to be administered. However, adipose tissue of the same kinds of animals (other animals) or adipose tissue heterogeneous animals may be used.

An example of adipose tissue can include subcutaneous fat, offal fat, intramuscular fat, and inter-muscular fat. Among them, subcutaneous fat is a preferable cell source because it can be collected under local anesthesia in an extremely simple and easy manner and therefore the burden to a doner in collection is small. In general, one kind of adipose tissue is used, but two kinds or more of adipose tissues can be used. Furthermore, adipose tissues (which may not be the same kind of adipose tissue) collected in a plurality of times may be mixed and used in the later operation. The collection amount of adipose tissue can be determined by considering the kind of donors or kinds of tissue, or the necessary amount of ASCs. For example, the amount can be from 0.5 g - 500 g. It is preferable that the collection amount at one time is about 10g - 20g or less by considering a burden to the donor. The collected adipose tissue is subjected to removal of blood components attached thereto and stripping if necessary and thereafter, subjected to the following enzyme treatment. Note here that by washing adipose tissue with appropriate buffer solution or culture solution, blood components can be removed.

The enzyme treatment is carried out by digesting adipose tissue with protease such as collagenase, trypsin and Dispase. Such an enzyme treatment may be carried out by techniques and conditions that are known to a person skilled in the art (see, for example, R.I. Freshney, Culture of Animal Cells:A Manual of Basic Technique, 4th Edition, A John Wiley & Sones Inc., Publication). A cell population obtained by the above-mentioned enzyme treatment includes multipotent stem cells, endothelial cells, interstitial cells, blood corpuscle cells, and/or precursor cells thereof. The kinds or ratios of the cells constituting the cell population depend upon the origin and kinds of adipose tissue to be used.

### (2) Obtaining of Sedimented Cell Population (SVF Fraction:Stromal Vascular Fractions)

The cell population is then subjected to centrifugation. Sediments obtained by centrifugation are collected as sedimented cell population (also referred to as "SVF fraction" in this specification). The conditions of centrifugation are different depending upon the kinds or amount of cells. The centrifugation is carried out for example, at 800-1500 rpm for 1-10 minutes. Prior to the centrifugation, cell population after enzyme treatment can be subjected to filtration and tissue that has not been digested with enzyme contained therein can be removed.

The "SVF fraction" obtained herein includes ASCs. Therefore, the SVF fraction can be used for a co-culture with sperm. The kinds or ratio of cells constituting the SVF fraction depend upon the origin and kinds of adipose tissue to be used, conditions of the enzyme treatment, and the like. The characteristics of the SVF fraction are showed in the International Publication WO2006/006692A1.

### (3) Selective culture of adhesive cells (ASC) and recovery of cells

Other cell components (such as endothelial cells, stroma cells, hematopoietic cells, and precursor cells thereof) are contained in a SVF fraction other than ASC. Thus, in one embodiment of the present invention, unnecessary cell components are removed from the SVF fraction by carrying out the following selective culture. Then, cells that are obtained as a result are used in the present invention as ASC.

Firstly, a SVF fraction is suspended in an appropriate medium, and then seeded on a culture dish and cultured overnight. Floating cells (non-adhesive cells) are removed by replacement of a medium. Then, culture is continued while suitable replacement of a medium (for example, once per 2-3 days). Subculture is carried out according to necessity. The passage number is not particularly limited. However, it is not preferable to excessively run over the subculture from the view point of maintenance of pluripotency and proliferation potency (preferably up to the fifth passage). Note that, for the culture medium, a medium for normal animal cell culture can be used. Examples such as Dulbecco's modified Eagle's Medium (DMEM) (NISSUI PHARMACEUTICAL, etc.), α-MEM (Dainippon Seiyaku, etc.), DMED:Ham's F12 mixed medium (1:1) (Dainippon Seiyaku, etc.), Ham's F12 medium (Dainippon Seiyaku, etc.), and MCDB 201 medium (Research Institute for the Functional Peptides) can be used. Media added with serums (fetal bovine serum, human serum, sheep serum, etc.) or serum replacement s (Knockout serum replacement (KSR), etc.) may also be used. The adding amount of a serum or serum replacement can be set within the range from 5% (v/v) - 30% (v/v), for example.

Adhesive cells selectively survive and proliferate according to the above mentioned operations. Next, the cells proliferated are collected. The cells may be collected by routine procedures and, for example, collected easily by enzyme treatment (treatment with trypsin or Dispase) and then cells are scraped out by using a cell scraper, a pipette, or the like. Furthermore, when sheet culture is carried out by using a commercially available temperature sensitive culture dish, cells may be collected in a sheet shape without carrying out enzyme treatment. Use of thus collected cells (ASC) makes it possible to prepare a cell population containing ASC at high purity.

### (4) Low-serum culture (selective culture in a low-serum medium) and collection of cells

In one embodiment of the present invention, the following low-serum culture is carried out in place of or after (3) mentioned above. Then, the cells obtained as a result are used as ASC n the present invention.

In low-serum culture, the SVF fraction (when this step is carried out after (3), the cells that are collected in (3) are used) is cultured under the low-serum conditions and a desired multipotent stem cell (that is, ASC) is selectively proliferated. Since the amount of serum to be used is small in the low-serum culture method, in a case where the activated sperm obtained by the method of the present invention is used for the purpose of treatment, it is possible to use the serum of the subjects (recipients) themselves. That is to say, culture using autoserum can be carried. The "under low-serum conditions" herein denotes conditions in which a medium contains not more than 5% serum. Preferably, the sedimented cell population is cultured in a culture solution containing not more than 2% (V/V) serum. More preferably, the cells are cultured in a culture solution containing not more than 2% (V/V) serum and 1-100 ng/ml of fibroblast growth factor-2 (bFGF).

The serum is not limited to fetal bovine serum. Human serum, sheep serum, and the like, can be used. In a case where the activated sperm obtained by the method of the present invention is used for treatment of human, preferably, the human serum, more preferably the serum of a subject of the treatment (that is to say, autoserum) is used.

As the medium, a medium for culturing animal cells can be used on condition that the amount of serum contained in the use is low. For example, Dulbecco's modified Eagle's Medium (DMEM) (NISSUI PHARMACEUTICAL, etc.), α-MEM (Dainippon Seiyaku, etc.), DMED:Ham's:F12 mixed medium (1:1) (Dainippon Seiyaku etc.), Ham's F12 medium (Dainippon Seiyaku, etc.), MCDB201 medium (Research Institute for the Functional Peptides), and the like, can be used.

By culturing by the above-mentioned method, multipotent stem cells (ASCs) can be selectively proliferated. Furthermore, since the multipotent stem cells (ASCs) proliferated in the above-mentioned culture conditions have a high proliferation activity, it is possible to easily prepare cells necessary in number for the present invention by subculture. Note here that the characteristics of the cells selectively proliferated by low-serum culture of SVF fraction are shown in the International Publication WO2006/006692A1.

Subsequently, selectively proliferated cells by the above-mentioned low-serum culture are collected. A collection operation may be carried out in the same manner as in the case of (3). Use of thus collected cells (ASC) makes it possible to prepare a cell population containing ASC at high purity.

In the above-mentioned method, the cells proliferated by low-serum culture of SVF fraction is used for the present invention. However, cells proliferated by the low serum culture of cell population obtained from adipose tissue (without carrying out centrifugation for obtaining SVF fraction) can be used as ASCs. That is to say, in one embodiment of the present invention, cells proliferated by the low-serum culture of cell population obtained from adipose tissue are used as low-serum culture ASCs. Not multipotent stem cells that are obtained according to selective culture ((3) and (4) mentioned above) but a SVF fraction (containing adipose tissue-derived mesenchymal stem cells) can be directly used. Note that "directly used" herein means that a SVF fraction is used in the present invention without undergoing selective culture.

### <Applicable diseases and administration method>

The therapeutic agent of the present invention is used for treating and preventing ED. Therefore, the therapeutic agent of the present invention will usually be administered to patients with ED. However, the therapeutic agent of the present invention can be also used for the purpose of experiment or research such as confirming and verifying the effect.

Although any of organic, psychogenic and mixed ED can be an object to be treated, the therapeutic agent of the present invention is preferably used for the treatment of organic (particularly, neurogenic, vascular or diabetic) erectile disorder or mixed erectile disorder.

The existing drug, PDE-5 inhibitor, inhibits degradation of cyclic GMP to help relax cavernous smooth muscle of the penis and promote erection. PDE-5 inhibitors are generally not effective enough for organic ED such as vascular ED, neurogenic ED and diabetic ED. In addition, PDE-5 inhibitors have systemic effects and may have side effects such as hot flashes, headaches, and flushing. The therapeutic agent of the present invention can solve these problems of PDE-5 inhibitors, and thus has great clinical significance and utility value.

The therapeutic agent of the present invention is preferably administered by local injection into the affected area. The site of injection is typically the corpus cavernosum penis or corpus spongiosum penis. However, it may be injected into the external urethral sphincter or under the urethral mucosa of the external urethral sphincter part. Moreover, the administration may be performed at two or more injection sites simultaneously or at time intervals.

The dose (injection amount) of the therapeutic agent of the present invention is, for example, 0.5 ml to 10 ml, and preferably 1 ml to 5 ml. It is advisable to administer multiple doses while changing the injection site, instead of administering the entire dose in a single injection.

The administration schedule may be prepared in consideration of the subject's (patient's) sex, age, weight, pathological condition, and the like. In addition to a single dose, multiple doses may be administered continuously or periodically. The administration interval when administering multiple doses is not particularly limited and is, for example, 1 day to 1 month. Moreover, the number of administrations is not also particularly limited. Examples of the number of administrations are 2 to 10 times.

When applying the therapeutic agent of the present invention, an existing drug (e.g., PDE-5 inhibitor, prostaglandin preparation) may be co-administered. That is, an existing drug may be used in combination with the therapeutic agent of the present invention. Such combined use can be expected to increase the therapeutic effect. Examples of the PDE-5 inhibitor are sildenafil citrate tablets (trade name: Viagra tablets), vardenafil hydrochloride hydrate tablets (trade name: Levitra tablets) and tadalafil (trade name: Cialis tablets), and an example of the prostaglandin preparation is prostaglandin E1 preparation (trade name: prostaglandin for injection).

### [Examples]

### 1. Preparation of stem cell filtrate

### (1) Preparation of ASC filtrate

Human ASC was prepared from subcutaneous fat by a conventional method, and after adjusting the concentration (1 × 10⁶ cells/ml PBS), it was stored at -30°C for one or more nights (stored at -80°C when not used immediately). The cell liquid was thawed in warm water at 38°C or at room temperature. After disrupting the cells in this manner, centrifugation (1200 rpm, 5 minutes) was performed and the supernatant was collected. Next, the supernatant was filtered through a cellulose acetate membrane filter (pore size 0.2 µm) to obtain an ASC filtrate.

### (2) Preparation of BM-MSC filtrate (freeze-thaw disruption)

Human bone marrow-derived stem cells (BM-MSCs) prepared by a conventional method and stored frozen were thawed in warm water at 38°C or at room temperature, and then centrifuged (1200 rpm, 5 minutes). The supernatant was filtered through a cellulose acetate membrane filter (pore size 0.2 µm) to obtain a BM-MSC filtrate (freeze-thaw disruption).

### (3) Preparation of BM-MSC filtrate (ultrasonic disruption)

Human bone marrow-derived stem cells (BM-MSC) prepared by a conventional method and stored frozen were sonicated (250 W output, repeating 10 seconds of disruption and 20 seconds of rest for 30 minutes) (using BIORUPTOR (UCD-250) from Cosmo Bio Co., Ltd.), followed by centrifugation (1200 rpm, 5 minutes). The supernatant was filtered through a cellulose acetate membrane filter (pore size 0.2 µm) to obtain a BM-MSC filtrate (ultrasonic disruption).

### 2. Effect of stem cell filtrate on vascular ED model

For 8-week-old, male, Wistar-ST rats (purchased from SLC), an incision was made in the lower abdomen under isoflurane anesthesia (induction 3%, maintenance 1.5% to 2%), and the internal iliac artery was identified and double-ligated with thread to create a vascular ED model in which blood inflow into the corpus cavernosum penis was blocked. As the control group, rats subjected to sham operation with only abdominal suture were used. Immediately after the ligation operation, the ASC filtrate (100 µl) or vehicle (PBS 100 µl) was injected into the corpus cavernosum penis of the vascular ED model. Erectile function was evaluated 4 weeks after the operation (after the administration of filtrate). The erectile function was evaluated using the intracavernosal pressure measurement method. Under isoflurane anesthesia (induction 3%, maintenance 1.5% to 2%), systemic blood pressure was monitored from the left carotid artery and intracavernosal pressure was monitored from the crura penis. The cavernous nerve was identified and electrically stimulated (5 V, pulse width 5 msec, 1, 2, 4, 8, 16 Hz) with bipolar electrodes, and fluctuation was recorded. A value obtained by dividing the intracavernosal pressure by the mean blood pressure (ICP/MAP) was used as the erectile function. In the vascular ED + PBS group (Ligation + PBS group), the ICP/MAP was decreased as compared to that in the control Sham group, and a decrease in erectile function was observed (Fig. 1). On the other hand, in the vascular ED + adipose stem cell filtrate group (Ligation + Adipose group), the ICP/MAP value was higher than that in the Ligation + PBS group, and improvement of erectile function was observed (Fig. 1).

### 3. Effect of stem cell filtrate in diabetic ED model

For 8 week-old male Wistar-ST rats (purchased from SLC), 40 mg/kg of streptozotocin (STZ) was intraperitoneally administered under isoflurane anesthesia (induction 3%, maintenance 1.5% to 2%) to create a diabetic ED model. Blood glucose levels were measured on the first week after STZ administration, and only individuals with 250 mg/dL or more were used. The ASC filtrate (100 µl) or vehicle (PBS 100 µl) was injected into the corpus cavernosum penis in individuals who developed diabetes. The erectile function was evaluated 4 weeks after the cavernosal injection (after the administration of filtrate). In the STZ + PBS group, the ICP/MAP was decreased as compared to that in the control group (CP) and a decrease in erectile function was observed (Fig. 2). On the other hand, in the STZ + ASC filtrate group (STZ + Adipose group), the ICP/MAP value was high as compared to that in the STZ + PBS group, and improvement of erectile function was observed (Fig. 2).

### 4. Effect of stem cell filtrate in neurogenic ED model

For 8-week-old, male, Wistar-ST rats (purchased from SLC), the cavernous nerve was exposed under isoflurane anesthesia (induction 3%, maintenance 1.5% to 2%), and both sides were clamped with reverse action tweezers to create a bilateral cavernous nerve injury model (BCNI model). As the control group, rats subjected to sham operation were used. Immediately after the operation, the BM-MSC filtrate (freeze-thaw disruption) (100 µl) or vehicle (PBS 100 µl) was injected into the corpus cavernosum penis. The erectile function was evaluated on the fourth week after the administration. In the BCNI + PBS group, the ICP/MAP was significantly decreased as compared to that in the sham group, and a decrease in erectile function was observed. On the other hand, in the BCNI + BM-MSC filtrate group (BCNI + Bone group), the ICP/MAP was significantly improved as compared to that in the BCNI + PBS group and improvement of erectile function was observed (Fig. 3).

### 5. Effect of stem cell filtrate prepared by non-freezing disruption (ultrasonic disruption) (neurogenic ED Model)

For 8-week-old, male, Wistar-ST rats (purchased from SLC), the cavernous nerve was exposed under isoflurane anesthesia (induction 3%, maintenance 1.5% to 2%), and both sides were clamped with reverse action tweezers to create a bilateral cavernous nerve injury model (BCNI model). As the control group, rats subjected to sham operation were used. Immediately after the operation, the BM-MSC filtrate or vehicle (PBS) was injected into the corpus cavernosum penis. The filtrate was prepared by freeze-thaw disruption or ultrasonic disruption after stem cell collection, followed by filtering, as described above. The erectile function was evaluated on the second week after the administration. In the BCNI + PBS group, the ICP/MAP was decreased as compared to that in the sham group, and a decrease in erectile function was observed. On the other hand, in the BCNI + BM-MSC filtrate (freeze-thaw disruption) group (BCNI + BoneFoezn group) and the BCNI + BM-MSC filtrate (ultrasonic disruption) group (BCNI + BoneSonication group), the ICP/MAP was improved as compared to that in the BCNI + PBS group and improvement of erectile function was observed (Fig. 4). Also, at low stimulation frequencies of 2 Hz and 4 Hz, ultrasonic disruption (BCNI + BoneSonication group) showed a higher degree of improvement than freeze-thaw disruption (BCNI + BoneFoezn group) (Fig. 4).

### 6. Summary

As described above, it was demonstrated that the stem cell filtrate is extremely useful as a preventive or therapeutic drug for ED. Use of the stem cell filtrate, which is an acellular preparation, rather than the stem cells themselves, enables treatment with significantly higher safety than the previously reported stem cell treatment. In particular, when the stem cell filtrate is administered by cavernosal injection, the risk of systemic side effects is greatly reduced.

### [Industrial Applicability]

The therapeutic agent of the present invention is used for treating and preventing erectile dysfunction. The therapeutic drug of the present invention use a filtrate of the specific stem cells (obtained by filtering a disrupted cell solution) as an active ingredient, and shows efficacy by a different mechanism of action from the currently mainstream therapeutic drug (PDE-5 inhibitor). Therefore, it can be expected that the therapeutic effect is exerted even on patients for which conventional therapeutic methods have not been effective.

The invention is not limited by the description of the embodiments and examples of the invention described above at all. Various modified embodiments are also included in the invention within the range that a person skilled in the art can easily conceive of, without deviating from the scope of the claims. Contents of treatises, unexamined patent publications, and examined patent publications specified in this specification are all incorporated herein by reference.

## Claims

1. A therapeutic agent for erectile dysfunction, comprising a filtrate obtained by filtering a disrupted solution of adipose tissue-derived stem cells or bone marrow-derived stem cells.

2. The therapeutic agent for erectile dysfunction according to claim 1, wherein the disrupted solution is centrifuged before the filtering, and an obtained supernatant is filtered.

3. The therapeutic agent for erectile dysfunction according to claim 1 or 2, wherein the disrupted solution is obtained by ultrasonic treatment.

4. The therapeutic agent for erectile dysfunction according to any one of claims 1 to 3, which is used for treating organic erectile disorder or mixed erectile disorder.

5. The therapeutic agent for erectile dysfunction according to claim 4, wherein the organic erectile disorder is neurogenic, vascular or diabetic erectile disorder.

6. The therapeutic agent for erectile dysfunction according to any one of claims 1 to 5, which is used in combination with a PDE-5 inhibitor and/or a prostaglandin preparation.

7. A method for producing a therapeutic agent for erectile dysfunction, comprising following steps (1) to (3):
(1) disrupting adipose tissue-derived stem cells or bone marrow-derived stem cells;
(2) obtaining a filtrate by filtering a disrupted solution obtained in step (1), or a supernatant obtained by centrifuging the disrupted solution; and
(3) formulating the filtrate obtained in step (2).

8. The production method according to claim 7, wherein step (1) is performed by ultrasonic treatment.

9. A method for treating erectile dysfunction, comprising administering the therapeutic agent for erectile dysfunction according to any one of claims 1 to 5, to the corpus cavernosum penis, the corpus spongiosum penis, the external urethral sphincter or under the urethral mucosa of the external urethral sphincter part of a patient with erectile dysfunction.

10. The treatment method according to claim 9, wherein a PDE-5 inhibitor and/or a prostaglandin preparation is co-administered.
